Europäisches Patentamt

European Patent Office

Office européen des brevets

(11) Numéro de publication: **0 419 354 A2**

# DEMANDE DE BREVET EUROPEEN

(21) Numéro de dépôt: 90402588.9

(22) Date de dépôt: **19.09.90**

(51) Int. Cl.5: **A61K 31/70**

(30) Priorité: **20.09.89 FR 8912353**

(43) Date de publication de la demande:
**27.03.91 Bulletin 91/13**

(84) Etats contractants désignés:
**AT BE CH DE DK FR GB IT LI LU NL SE**

(71) Demandeur: **DELALANDE S.A.**
**32, rue Henri Regnault**
**F-92400 Courbevoie(FR)**

(72) Inventeur: **Petavy, Anne-Françoise, Lab. de**
**Parasitologie**
**Faculté de Pharmacie, 8, avenue Rockfeller**
**F-69373 Lyon Cedex 08(FR)**
Inventeur: **Sarciron, Elizabeth, Lab. de**
**Parasitologie**
**Faculté de Pharmacie, 8, avenue Rockfeller**
**F-69373 Lyon Cedex 08(FR)**
Inventeur: **Paris, Joelle Lab. de Parasitologie**
**Fac. de Pharmacie 8, avenue Rockefeller**
**F-69373 Lyon Cédex 08(FR)**

(74) Mandataire: **Kedinger, Jean-Paul et al**
**c/o Cabinet Malemont 42, avenue du**
**Président Wilson**
**F-75116 Paris(FR)**

(54) Utilisation d'un complexe de l'inosine avec un N,N-dialkylamino alcanol ou un sel de celui-ci avec un acide pharmacologiquement acceptable pour le traitement de certaines infections helminthiques.

(57) Utilisation des complexes de l'inosine avec un N,N-dialkylamino alcanol ou un sel de celui-ci avec un acide pharmacologiquement acceptable, pour la fabrication d'un médicament ou d'une composition pharmaceutique pour le traitement d'infections helminthiques.

EP 0 419 354 A2

## UTILISATION D'UN COMPLEXE DE L'INOSINE AVEC UN N,N-DIALKYLAMINO ALCANOL OU UN SEL DE CELUI-CI AVEC UN ACIDE PHARMACOLOGIQUEMENT ACCEPTABLE POUR LE TRAITEMENT DE CERTAINES INFECTIONS HELMINTHIQUES

La présente invention a pour objet une nouvelle utilisation thérapeutique des complexes de l'inosine avec un N,N-dialkylamino alcanol ou un sel de celui-ci avec un acide pharmacologiquement acceptable.

Les complexes de ce type sont bien connus pour leur activité immunostimulante et antivirale (brevet français No. 70 30995). En particulier, le complexe (3:1) p-acétamidobenzoate de N,N-diméthylamino isopropanol:inosine dont les structure, préparation, propriétés physico-chimiques et activités pharmacologiques sont décrites dans la littérature [(brevet français No. 70 30995 Fed. Proc. 29, (1970); Can. J. Microbiol. 18, 1463 (1972) ; Antimicrob. Ag. Chemother. 2, 224 (1972)] est largement utilisé en thérapeutique antivirale sous des marques de fabrique diverses et notamment en France sous la marque ISOPRINOSINE.

Il a maintenant été mis en évidence que, de manière tout à fait inattendue, les complexes susmentionnés s'avèraient également efficaces dans le traitement de certaines infections helminthiques telles que l'échinococcose alvéolaire et l'échinococcose hydatique.

L'échinococcose alvéolaire, l'une des infections helminthiques humaines les plus léthales, a fait l'objet d'une description détaillée dans Sem. Hôp. Paris, 2691-2701 (17 nov. 1988). Quant à l'échinoccose hydatique, une description détaillée en a été faite dans l'Am. J. Surg. en 1978 (Vol. 135, p. 597 et suivantes).

L'homme fait figure d'hôte intermédiaire accidentel des parasites Echinococcus multilocularis responsable de l'échinococcose alvéolaire et Echinococcus granulosus responsable de l'échinococcose hydatique. C'est surtout dans le foie que la larve de ces cestodes se développe. Longtemps quiescente, l'atteinte se manifeste long terme, comme une tumeur maligne pour Echinococcus multilocularis et sous une forme kystique pour Echinococcus granulosus. Des localisations pulmonaires et cérébrales sont également constatées.

La thérapeutique actuelle n'est que palliative et repose essentiellement sur l'exérèse chirurgicale. Par ailleurs, la chimiothérapie, représentée essentiellement par les dérivés benzimidazolés, n'a fait preuve que d'une activité larvicide limitée, bien qu'elle puisse, dans certains cas, ralentir l'évolution de ces affections.

Dès lors, il existait un besoin pressant de mise au point de substances capables d'exercer un effet léthal sur les larves d'Echinococcus multilocularis et d'Echinococcus granulosus et donc de combattre les infections en cause. La mise en évidence par la Demanderesse que les complexes de l'inosine avec un N,N-dialkylamino alcanol ou un sel de celui-ci avec un acide pharmacologiquement acceptable possédaient de telles propriétés, ouvre donc la voie une nouvelle méthode de traitement, efficace et dépourvue des inconvénients des thérapeutiques existant à ce jour.

L'un des objets de la présente invention réside par conséquent dans l'utilisation des complexes de l'inosine avec un N,N-dialkylamino alcanol ou un sel de celui-ci avec un acide pharmacologiquement acceptable, pour le traitement de certaines infections helminthiques telles que l'échinococcose alvéolaire et l'échinococcose hydatique.

A titre de N,N-dialkylamino alcanol, on pourra citer les composés dont le reste alcanol est choisi parmi les restes éthanol, n-propanol, isopropanol et butanol et dont le reste dialkylamino est choisi parmi les restes diméthylamino, diéthylamino, di(n-propyl)amino, diisopropylamino, di(n-butyl)amino et méthyléthylamino, le N,N-diméthylamino isopropanol présentant un intérêt tout particulier.

A titre d'acide pharmacologiquement acceptable, on citera les acides minéraux tels que les acides chlorhydrique, sulfurique et phosphorique et les acides organiques tels que les acides tartrique maléique, fumarique, succinique et p-acétamidobenzoïque, ce dernier étant particulièrement préféré.

Le rapport molaire entre le N,N-dialkylamino alcanol ou son sel et l'inosine peut varier de 1 à 10 et est de préférence 3.

Comme indiqué précédemment, les complexes ci-dessus sont notamment décrits dans le brevet français No. 70 30995 qui contient toutes informations utiles à leur préparation.

Il convient encore de noter que le complexe (3:1) p-acétamidobenzoate de N,N-diméthylamino isopropanol:inosine est présentement le complexe préféré selon l'invention. Sa toxicité est très faible (DL50 : 10000 - 15000 mg/kg/po chez la souris et 7500 -14700 mg/kg/po chez le rat). C'est ce complexe qui est utilisé à titre d'exemple dans l'étude décrite ci-après pour mettre en évidence l'activité anti-helminthique revendiquée.

Cette étude a été effectuée sur des gerbilles de Chine (Meriones ungisculatus) âgées de 6 mois. Les animaux ont été stabulés et alimentés dans des conditions standards.

Trois mois avant l'expérimentation, tous les animaux ont été infestés par l'inoculation intra-péritonéale de 50 mg de larve d'Echinococcus multilocularis, d'une souche isolée de Haute-Savoie entretenue par passages successifs sur gerbilles (YAMASHITA et coll., Jap. J. Vet. Res. 6, 136, 1958 CONTAT F., Thèse de médecine vétérinaire (1984), Université Claude Bernard - Lyon).

Le complexe d'essai a été administré par voie orale (gavage). Une dose unique quotidienne de traitement a été administrée pendant 5 jours consécutifs puis au treizième jour, sous un volume de 0,5 ml/animal.

Cinq lots d'animaux ont été constitués comme suit :

lot 1 : 5 animaux à chacun desquels ont été administrés 250 mg de principe actif par jour, sous forme de comprimé broyé (comprenant, outre ledit principe actif, un excipient constitué de mannitol, amidon de blé, polyvidone et stéarate de magnésium) mis en suspension aqueuse en présence de gomme arabique ;

lot 2 : 5 témoins recevant le même volume de véhicule (gomme arabique) seul ;

lot 3 : 3 animaux à chacun desquels ont été administrés 250 mg de principe actif par jour, sous forme du principe actif pur en mélange avec 0,5 ml de gomme arabique ;

lot 4 : identique au lot 1 pour confirmation des observations faites avec ce dernier ;

lot 5 : identique au lot 2.

Tous les animaux ont été suivis de manière identique : état général et poids corporel ont notamment été relevés.

Puis ils ont été sacrifiés au 19ème jour (par élongation cervicale).

Les larves d'Echinococcus multilocularis ont été extraites de la cavité abdominale par dissection : leur morphologie et leur poids ont été notés après élimination des tissus réactionnels environnants.

Un examen histologique en microscopie électronique des tissus larvaires a ensuite été effectué.

L'état général des animaux a été trouvé plus satisfaisant dans les lots traités par le complexe selon l'invention que dans les lots témoins, avec notamment une plus grande vivacité et un meilleur état du pelage.

Le poids des animaux est augmenté dans les lots traités (moyenne : 71,50 ± 2,31 g) par rapport à ceux constatés dans les lots témoins (moyenne : 65,11 ± 2,51 g) et ce, malgré une diminution du poids des larves.

Macroscopiquement, les larves sont toujours apparues de taille réduite et de couleur plus sombre chez les animaux traités que chez les témoins.

Par ailleurs, comme le montre le tableau ci-après, le poids des larves s'est révélé significativement inférieur dans les lots traités que dans les lots témoins.

En microscopie électronique, les lésions observées sur les larves sont plus importantes dans les lots 1, 3 et 4 que dans les lots témoins.

Tableau : poids des larves

| Animal | Lot 1 | Lot 2 | Lot 3 | Lot 4 | Lot 5 |
|--------|-------|-------|-------|-------|-------|
| No. 1 | 6,75 | 10,30 | 8,13 | 6,50 | 10,41 |
| No. 2 | 6,50 | 10,20 | 7,48 | 6,28 | 10,31 |
| No. 3 | 6,83 | 10,52 | 8,21 | 6,71 | 10,58 |
| No. 4 | 6,70 | 10,80 | - | 6,48 | 10,51 |
| No. 5 | 6,71 | 9,80 | - | 6,39 | 10,52 |
| $m \pm et$ | $6,7 \pm 0,12$ | $10,34 \pm 0,39$ | $7,94 \pm 0,40$ | $6,47 \pm 0,16$ | $10,47 \pm 0,11$ |
| Stat. | $p < 0,001$ | | $p < 0,001$ | $p < 0,001$ | |
| | | | $p < 0,001$ | | |

m = moyenne
et = écart type
stat. = interprétation statistique (test t)

Ce qui précède montre que le traitement des gerbilles de Chine infestées par Echinococcus multilocularis par le complexe (3:1) p-acétamidobenzoate de N,N-diméthylamino isopropanol:inosine dans les conditions sus-dites, a entraîné une atteinte morphologique importante des larves, constatée macroscopiquement et en microscopie électronique.

Par ailleurs, ces effets sont apparus dans un temps plus court et les atteintes des larves sont plus importantes que celles observées avec les dérivés benzimidazolés considérés actuellement comme les seuls agents de traitement de la maladie.

Une autre étude a été effectuée chez la souris infestée 8 mois auparavant par Echinococcus granulosus . Cette infestation a été réalisée chez des souris âgées de 6 semaines, par injection intrapéritonéale de 4 000 protoscolex provenant de liquide hydatique de poumons de mouton obtenus à l'abattoir. La viabilité des protoscolex a été vérifiée avant l'inoculation aux souris.

Deux types de traitements ont été mis en oeuvre :

1°/ Traitement durant 5 jours consécutifs (J1 à J5) puis à J13, avec sacrifie à J19.

- 3 lots d'animaux ont été réalisés :

lot 1a : 4 animaux à chacun desquels il a été administré 1 g/kg de poids corporel de principe actif par jour, sous forme de comprimé broyé de même composition que lors de l'étude sur Echinococcus multilocularis , mis en suspension aqueuse en présence de gomme arabique ;

lot 2a : 4 animaux recevant 2 g/kg/jour de principe actif comme ci-dessus ;

lot 3a : 5 animaux témoins recevant le même volume de véhicule (gomme aratique) seul.

2°/ Traitement d'une semaine sur deux pendant 2 mois avec sacrifice le lendemain du dernier jour de traitement.

- 3 lots d'animaux ont été constitués :

lot 1b : 6 animaux, même dose journalière que le lot 1a ;

lot 2b : 6 animaux, même dose journalière que le lot 2a ;

lot 3b : 5 animaux témoins, comme le lot 3a.

Tous les animaux ont été suivis de manière identique : mortalité, examen macroscopique à l'autopsie, examen des kystes en microscopie électronique.

Les deux types de traitement ayant donné des résultats similaires, les résultats ont été regroupés.

Dans les lots traités, la mortalité a été soit nulle, soit plus réduite que chez les témoins :

- lot 1a + 1b = 1 mort sur 10
- lot 2a + 2b = 0 mort sur 10

- lot 3a + 3b = 3 morts sur 10

A l'autopsie, alors que les 7 témoins survivants (lots 3a et 3b) présentaient une rate volumineuse, 5 animaux sur les 9 survivants des lots 1a + 1b et 3 animaux sur 10 dans les lots 2a + 2b avaient une rate de volume intérieur aux témoins ou de volume normal.

Alors que tous les témoins (lots 3a et 3b) avaient un envahissement important ou complet de la cavité abdominale et du foie par des kystes hydatiques volumineux accompagnés de petits kystes souvent multiples, 3 animaux sur les 9 survivants des lots 1a + 1b et 3 animaux sur 10 des lots 2a + 2b n'avaient plus qu'une infestation légère ou nulle par le parasite.

Une modification de l'aspect normalement translucide des kystes hydatiques a été observée :
- chez un témoin sur 7, où l'un des kystes avait un aspect caséeux,
- chez 6 animaux sur 9 des lots 1a + 1b et 5 animaux sur 10 des lots 2a et 2b où un ou plusieurs kystes étaient opacifiés ou caséeux.

En microscopie électronique, les kystes provenant des animaux des lots traités présentaient des signes de dégénérescence : épaississement et hypervésiculation du syncitium tégumentaire superficiel pouvant aller jusqu'à une perte d'intégrité tégumentaire, dégénérescence du parenchyme avec hypervésiculation, raréfaction et/ou disparition des éléments cellulaires, présence de structures lamellaires, présence de grandes quantités de lipides, altération des cellules musculaires.

Il apparaît ainsi que le complexe (3:1) p-acétamidobenzoate de N,N-diméthylamino isopropanol:inosine en particulier et les complexes de l'inosine avec un N,N-dialkylamino alcanol ou un sel de celui-ci avec un acide pharmacologiquement acceptable en général, possèdent une activité toxique sans précédent sur les larves d'Echinococcus multilocularis et d'Echinococcus granulosus et, de ce fait constituent un moyen de choix pour le traitement d'infections helminthiques telles que l'échinococcose alvéolaire et l'échinococcose hydatique.

Un autre objet de la présente invention réside dans l'utilisation des complexes susmentionnés pour la préparation de médicaments pour le traitement de certaines infections helminthiques telles que l'échinococcose alvéolaire et l'échinococcose hydatique et notamment de compositions pharmaceutiques comprenant au moins l'un de ces complexes en association avec un support ou un excipient physiologiquement acceptable.

Ces compositions peuvent par exemple être formulées en vue de leur administration orale ou injectable.

Pour l'administration par voie orale, lesdites compositions peuvent notamment prendre la forme de comprimés, dragées, gélules, poudres ou granulés préparés par les techniques habituelles utilisant des supports et excipients connus tels que des diluants (par exemple, polyéthylène glycol, mannitol, lactose et dextrose), des liants (par exemple, amidon, gomme arabique, gélatine, méthylcellulose, carboxyméthyl cellulose et polyvidone), des charges, des lubrifiants (par exemple, stéarate de magnésium ou de calcium, talc et silice) et des agents de désintégration (par exemple, amidon, alginate).

Pour l'administration par voie orale, les compositions selon l'invention peuvent également prendre la forme de solutions, suspensions ou sirops.

Pour l'administration par voie injectable, les compositions selon l'invention peuvent notamment prendre la forme de solutions injectables comprenant un véhicule aqueux (eau stérile ou solutions aqueuses salines isotoniques stériles).

La dose à laquelle les principes actifs, c'est-a-dire les complexes susmentionnés, peuvent être administrés à l'homme, dépend de divers paramètres et entre autres de la voie d'administration, de la puissance thérapeutique des complexes, du poids corporel et de l'état pathologique du patient, ainsi que du stade évolutif de la maladie. Généralement, par voie orale, les doses quotidiennes (en une ou plusieurs prises) pourront se situer entre 50 et 500 mg/kg de poids corporel), étant bien précisé que cet intervalle n'est nullement limitatif, les doses efficaces pouvant, en considération des paramètres ci-dessus, se situer à l'extérieur dudit intervalle.

Dans un but d'illustration de la présente invention, on donne ci-après deux exemples de compositions pharmaceutiques réalisées par mise en oeuvre des complexes mentionnés et définis ci-dessus.

Exemple 1 : Préparation de comprimés

|  | par comprimé |
|---|---|
| Complexe (3:1) p-acétamidobenzoate de N,N-diméthylaminoisopropanol : inosine | 500 mg |
| Mannitol | 67 mg |
| Amidon de blé | 67 mg |
| Polyvidone excipient | 10 mg |
| Stéarate de magnésium | 6 mg |

Introduire le complexe puis le mannitol et l'amidon de blé dans un mélangeur approprié. Mélanger. Mettre par ailleurs la polyvidone en solution dans de l'alcool dénaturé. Verser cette solution sur la masse en mouvement. Malaxer. Granuler sur toile grossière. Sécher. Granuler sur toile fine. Introduire le grain et le stéarate de magnésium dans un mélangeur. Mélanger. Comprimer dans une machine à comprimer.

Exemple 2 : Préparation d'une solution injectable par voie intraveineuse

|  | par ampoule |
|---|---|
| Complexe (3:1) p-acétamidobenzoate de N,N-diméthylaminoisopropanol : inosine | 500 mg |
| Eau pour préparations injectables q.s.p. | 5 ml |

Dissoudre sous agitation le complexe dans la presque totalité de l'eau pour préparations injectables. Compléter au volume avec de l'eau pour préparations injectables. Filtrer. Répartir en ampoules de 5 ml. Stériliser à 120° C pendant 30 minutes.

**Revendications**

1. Utilisation des complexes de l'inosine avec un N,N-dialkylamino alcanol ou un sel de celui-ci avec un acide pharmacologiquement acceptable, pour la fabrication d'un médicament ou d'une composition pharmaceutique pour le traitement d'infections helminthiques.

2. Utilisation selon la revendication 1, pour la fabrication d'un médicament ou d'une composition pharmaceutique pour le traitement de l'échinococcose alvéolaire et de l'échinococcose hydatique.

3. Utilisation selon la revendication 1 ou 2, du complexe de l'inosine avec le N,N-diméthylamino isopropanol ou un sel de celui-ci avec un acide pharmacologiquement acceptable.

4. Utilisation selon la revendication 1 ou 2, du complexe de l'inosine avec le p-acétamidobenzoate de N,N-diméthylamino isopropanol.

5. Utilisation selon la revendication 1 ou 2, du complexe (3:1) p-acétamidobenzoate du N,N-diméthylamino isopropanol:inosine.

6. Utilisation selon l'une quelconque des revendications précédentes, pour la fabrication d'une composition pharmaceutique formulée en vue de son administration par voie orale ou injectable.